# EUROPÄISCHE PATENTSCHRIFT

(11) **EP 1 311 850 B1**
(45) Veröffentlichungstag und Bekanntmachung des Hinweises auf die Patenterteilung: **07.05.2008**
(21) Anmeldenummer: 01954015.2
(22) Anmeldetag: 06.07.2001
(51) Int. Cl.: G01N 33/50, C12M 1/34, C12Q 1/02, G01N 13/00, G01L 1/00

(54) **VORRICHTUNG UND VERFAHREN ZUR MESSUNG VON KRÄFTEN VON BELEBTEM MATERIAL**
DEVICE AND METHOD FOR THE MEASUREMENT OF FORCES FROM LIVING MATERIALS
DISPOSITIF ET PROCEDE DE MESURE DE FORCES PRODUITES PAR DES MATIERES VIVANTES

(30) Priorität: 26.08.2000 DE 10041988
(43) Veröffentlichungstag der Anmeldung: 21.05.2003
(73) Patentinhaber: Fachhochschule Aachen, 52066 Aachen (DE)
(72) Erfinder: Artmann, Gerhard, Prof. Dr., 6291 HX Vaals (NL)
(74) Vertreter: Fitzner, Uwe
(86) Internationale Anmeldenummer: PCT/EP2001/007762
(87) Internationale Veröffentlichungsnummer: WO 2002/018937

(56) Entgegenhaltungen:
- DE-A- 4 125 398
- US-A- 5 181 416
- US-A- 5 284 753
- US-A- 6 044 717

## Beschreibung

Die vorliegende Erfindung betrifft ein Verfahren zur Messung von lateralen intrinsischen Kräften von belebtem Material sowie die Verwendung einer Vorrichtung zur Durchführung des Verfahrens.

Zellkräfte sind mechanische Kräfte , die von Zellen ausgehen und auf andere Zellen (interzellulär) oder Substrate übertragen werden. Die Kräfte werden innerhalb von Zellen (intrazellulär) generiert und außerhalb der Zellen auf benachbarte Zellen oder über die von den Zellen sekretierte extrazelluläre Matrix (EZM) auf ein Substrat übertragen. Solche Substrate können natürliche oder künstliche Oberflächen sein, wie z. B. Knochen, nicht-zelluläre Bindegewebsstrukturen, künstliche Implantate oder Biomaterialien. Die biologischen Elemente der Kraftentstehung und - fortleitung sind die Proteine. Neben dem osmotischen Innendruck des Cytosols, sind es die vernetzten Strukturproteine (Cytoskelett), die Kräfte innerhalb der Zellen entstehen lassen und durch die Zelle hindurch weiterleiten. Diese Kräfte werden über integrale Membranproteine, die mechanisch an den extrazellulären Raum gekoppelt sind, an Nachbarzellen oder andere Substrate weitergeleitet.

Die Zellen und die von ihnen ausgehenden Kräfte können durch unterschiedlichste Stimulanzien gereizt werden. Dadurch verändern sich die Strukturproteine und es kommt zu einer Verstärkung (Induktion) oder Verminderung (Relaxation) der Zellkräfte. Dies kann zu Kontraktionen, einer Bewegung in Richtung eines physikalischen oder chemischen Stimulus oder anderen Phänomenen führen. Die zugrundeliegenden Mechanismen sind nur teilweise bekannt. Makroskopische Auswirkungen sind z. B. Muskelkontraktion, Gefäßkontraktion oder -dilatation, Zellpassage durch das Gewebe oder beispielsweise die Haftung oder Ablösung der Zellen an bzw. von künstlichen Biomaterialien. Beides kann bei Biomaterialien je nach Anwendungsfall gewünscht sein.

Die Bestimmung von zellulären Kräften wurde bislang an isolierten Einzelzellen vorgenommen (J. van Velden et al.: Force production in mechanically isolated cardiac myocytes from human ventricular muscle tissue, Cardiovascular Research 38 (1998) 414-423). Die eingesetzten Zellen, hier Myocyten, waren dabei an dünne rostfreie Stahlnadeln mit Silikonkleber angeklebt (Spitzendurchmesser ∼ 15 µm). Dies kann undefinierbare Präparationsartefakte auslösen, die das Meßergebnis gegenüber dem tatsächlichen Zustand in den Zellen verfälschen. Ferner ist die Messung der Zellkraft hier sehr aufwendig und erfolgt durch die Verwendung eines Kraftüberträgers oder sogenannten "force transducer" (SensoNor, Horten, Norway) und eines piezoelektrischen Motors (Physik-Instrumente, Waldbrunn, Germany) sowie einer dünnen quadratischen Carbon-Faser (Länge 15 mm, Dicke 0,5 mm) zur Erreichung einer hinreichenden Empfindlichkeit.

Kolodney, M.S., et al. (1992, Isometric contraction by fibroblasts and endothelial cells in tissue culture: a quantitative study. J Cell Biol 117:73-82) beschreiben ein Verfahren bei dem einzelne Zellen in dreidimensionale Proteingele (Collagen-Gel-Netzwerk) einwachsen, wobei die Proteingele zwischen zwei Haltern gehalten werden. Die von den Zellen durch Anwachsen, Bewegung und/oder mechanische Aktivität in den dreidimensionalen Gelen hervorgerufenen Zugkräfte werden mit Hilfe eines Sensors bestimmt. Als Kraftsensor werden Dehnmeßstreifen verwendet, die ein elektrisches Signal erzeugen, durch das auf die Zugkraft zurückgeschlossen werden kann. Nachteilig bei diesem Verfahren ist jedoch, daß lediglich aus der Mechanik bekannte einfache Zugversuche nachgestellt werden. Die Anordnung erzeugt Querkontraktionen, wobei sich die Dicke des Proteingels entlang der Achse des Zugweges ändert. Gleichzeitig ändert sich jedoch auch die Spannungsverteilung in dem Gel. Dabei ist der Spannungszustand in dreidimensionalen Strukturen, wie der hier erwähnten Collagenmatrix weitgehend ortsabhängig. D. h., die mit Hilfe des Sensors gemessene Kraft ist abhängig von den Zellkräften an sich, aber auch von der Zellzahl, der Zellorientierung, dem Messzeitraum, da Zellen auf Spannungsgradienten durch Migration reagieren können, der Homogenität der Zellverteilung, der Nährstoffversorgung der Zellen durch die Martrix hindurch sowie der Geometrie und weitgehend unbekannten physikalischen Eigenschaften der Collagenmatrix, in der die Zellen eingebettet sind. Diese lokale, aber in einer dreisimensonalen Matrix unbekannte, Spannung, welche eine einzelne Zelle in der Matrix erfährt, führt zu einer uneinheitlichen und zeitlich veränderlichen Reaktion der Zellen, in Abhängigkeit von ihrem Lokalisationsort. D.h. die auf die einzelne Zelle einwirkenden, wechselnden Spannungen erlauben zwar eine reproduzierbare Messung der Zellkräfte in der Matrix, jedoch handelt es sich lediglich um durchschnittliche oder relative Kraftmessungen, vorausgesetzt die Messung erfolgt immer mit der gleichen Meßanordnung. Aussagen über die in einem zusammenhängenden biologischen System (Zellschichten) vorliegenden Kräfte können mit dem von Kolodney et al. offenbarten Experimenten nicht getroffen werden. Darüber hinaus ist das hier beschriebene Verfahren, auf die Untersuchung bestimmter Zellen beschränkt, da nicht alle Zellen ausnahmslos in einer dreidimensionalen Matrix, sondern ausschließlich als zelluläre Monolayer (oberflächlich) wachsen, wie z. B. Endothel- oder Epithelzellen.

Die US 5,181,416 betrifft eine Vorrichtung zur Bestimmung der Schärfe einer Kanüle. Dazu wird die Kraft bestimmt, die benötigt wird, um mit einer Kanüle eine Kuststofffolie zu durchbohren. Es handelt sich mithin nicht um eine Vorrichtung zur Messung von belebtem Material. Es wird auch nicht eine elastisch verformbare Membran, dass heißt eine durch Anlegen einer physikalischen Kraft elastisch verformbare Membran beschrieben, die mit dem belebten Material verbindbar ist, nachdem es auf diese Membran aufgebracht ist.

In der DE 4125398 A1 wird ein Drucksensor verbunden mit einer Membran enthaltend Bereiche unterschiedlicher Dicke beschrieben, der eine mechanische Kraft auf die Membran in eine elektrische Größe umwandelt. Auch hieraus ist nicht ersichtlich, dass eine Membran zur Messung von belebtem Material verwendet wird, insbesondere ist eine Verbindung von belebtem Material mit dieser Membran aus dieser Entgegenhaltung nicht erkennbar.

Einen Drucksensor basierend auf einem ähnlichen Prinzip wie in der DE 4125398 A1 geschildert, ist auch Gegenstand der US 6,044,717. Die Verbindung von belebtem Material mit einer Membran, die verformbar ist, kann dieser Entgegenhaltung nicht entnommen werden.

In der US 5,284,753 werden Membraneinsätze für Zellkulturen zur Untersuchung der Chemotaxis beschrieben. Dieser Druckschrift ist nicht eine Vorrichtung zur Messung von Kräften von belebtem Material zu entnehmen. Dass heißt, es wird nicht beschrieben eine verformbare Membran, die mit belebtem Material verbindbar ist, nachdem es auf diese Mebran aufgebracht ist.

Es sind somit keine Meßmethoden bekannt, welche die Bestimmung zellulärer Kräfte in zusammenhängenden Zellschichten oder sogar Zellgeweben oder ganzen Organen erlauben. Leibner, Th. et al (Abstract in Proceedings of the 4^{th} International Conference on Cellular Engeneering, Nara, Japan, 30.11-03.12.1999) geben zwar Hinweise darauf, daß es möglich ist, die von einer Zellschicht menschlicher Fibroblasten ausgehenden Kräfte zu messen, eine spezielle Apparatur oder eine genaue Vorgehensweise sind hier allerdings nicht offenbart.

Ferner ist die Quantifizierung von durch die Zellkommunikation bewirkten Zellkräften, wie z. B. ein synchrones Kontrahieren mehrerer Zellen oder eine von den Zellen erzeugte Autokontraktion bzw. -relaxation mit den bislang bekannten Verfahren nicht möglich.

Aufgabe der vorliegenden Erfindung ist es daher, ein Verfahren zur Bestimmung von lateralen intrinsischen Kräften in zusammenhängenden Zellschichten/ -geweben oder sogar (kultivierten) Organen zur Verfügung zu stellen, welche die zuvor genannten Nachteile nicht aufweisen. Darüber hinaus wäre ein Meßverfahren wünschenswert, mit dem in einem einzigen Versuchsansatz, d.h. an den gleichen Zellschichten, die biologischen Vorgänge (z. B. Veränderungen in der Zusammensetzung der Zellmatrix oder des Zytoskeletts oder Änderungen der Genexpression u.a.) beispielsweise aufgrund der Kultivierungsbedingungen direkt mit den Zellkräften korreliert werden könnten.

Diese Aufgabe wird durch die vorliegende Erfindung in vorteilhafter Weise gelöst, nämlich durch ein Verfahren zur Messung von Kräften von belebtem Material, wobei lateral wirkende intrinsische Kräfte des belebten Materials, ggf. vor, während und/oder nach erfolgter Stimulation des Materials durch externe Reize, direkt auf eine elastisch verformbare Membran übertragen werden und die hieraus resultierende Änderung der Durchbiegung der Membran quantitativ erfaßt wird. D.h., durch das vorliegende erfindungsgemäße Verfahren ist eine direkte Korrelation (und Quantifizierung) zwischen biologischen Vorgängen in Zellsichten (z. B.

Veränderung in der Zusammensetzung der Matrix, des Zytoskeletts oder der Genexpression) und den damit einhergehenden Änderungen der Zellkräfte möglich.

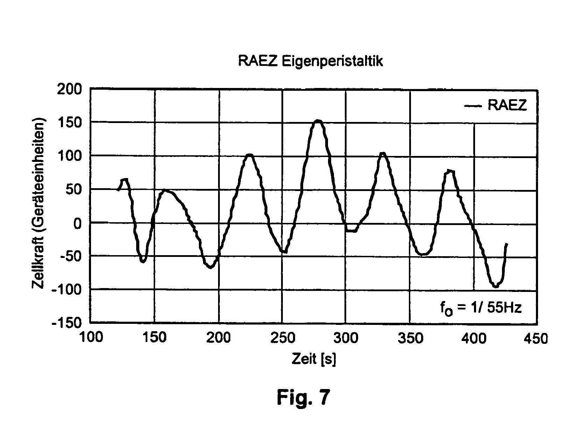

Unter intrinsischen Kräften sind erfindungsgemäß sowohl intrazelluläre als auch interzelluäre Kräfte zu verstehen. Dabei können erfindungsgemäß auch sogenannten rhythmische Eigenkontraktionen und/oder -relaxationen von Zellen bzw. Zellschichten gemessen werden. So zeigen z. B. Endothelzellen im Monolayerverband rythmische Eigenkontraktionen und - relaxationen, ohne daß die Zellen auf irgendeine Weise stimuliert wurden. Eine graphische Darstellung der Eigenperistaltik von Rinderaorten-Endothelzellen gemessen mit dem erfindungsgemäßen Verfahren ist in Fig. 7 dargestellt.

Im weiteren zeichnet sich das erfindungsgemäße Verfahren dadurch aus, daß eine elastisch verformbare Membran an einer Halterung plan gespannt wird, so daß sie von beiden Seiten frei zugänglich ist, anschließend die Membran durch das Anlegen einer physikalischen Kraft elastisch verformt wird, auf die elastisch verformte Membran belebtes Material aufgebracht und beispielsweise durch Kultivierung des belebten Materials und/oder eine weitere Haftvermittlung in und/oder auf der Membran, eine Verbindung zwischen dem belebten Material und der Membran ausgebildet wird. Darüber hinaus wird gegebenenfalls das belebte Material während und/oder nach der Ausbildung der Verbindung mit der Membran konstanten und/oder pulsierend und/oder schwingend angelegten externen Kräften ausgesetzt, ferner werden die von dem belebten Material ausgehenden lateralen intrinsichen Kräfte auf die Membran übertragen, gegebenenfalls zusätzliche externe Reize auf das belebte Material ausgeübt und die auf die Membran übertragenen Kräfte und/oder Kraftänderungen des belebten Materials, die damit verbundenen Zeitkonstanten und/oder Relaxationszeiten kontinuierlich als Veränderungen der Durchbiegung der Membran mit Hilfe eines Sensors quantitativ bestimmt.

Im Zuge des erfindungsgemäßen Verfahrens wird die Membran während und/oder nachdem sie plan gespannt wird/ist mit der Halterung verklebt und anschließend durch die Überschichtung mit einer Flüssigkeitssäule elastisch verformt, resultierend in einer Durchbiegung der Membran. Die Dehnung der Membran ist dabei vernachlässigbar. Bevorzugt entspricht der erfindungsgemäß an die Membran angelegte hydrostatische Druck bei einem Elastizitätsmodul von etwa 3900 MPa einer Flüssigkeitssäule mit einer Füllstandshöhe im Bereich von etwa 0,1 bis 50 mm, bevorzugt von etwa 0,5 bis 10 mm und besonders bevorzugt von etwa 2 mm. Beispielsweise entspricht bei einem Elastizitätsmodul von etwa 1000 MPa die Flüssigkeitssäule einer Füllstandshöhe von etwa 0,1 bis 10 mm und bei einem Elastizitätsmodul von etwa 10000 MPa einer Flüssigkeitssäule mit einer Füllstandshöhe von etwa 1 bis 150 mm. Allgemein verändern sich die anzulegenden Füllstandshöhen entsprechend den bekannten Gesetzen der Mechanik.

Bei der zuvor erwähnten Flüssigkeitssäule handelt es sich beispielsweise um ein zur Kultivierung des belebten Materials geeignetes Medium. Im Anschluß an die Verformung der Membran durch das Anlegen einer physikalischen Kraft durch Überschichten mit (Kultur)-Flüssigkeit, wird das belebte Material dann in dem Kulturmedium und auf der Membran kultiviert. Nachfolgend erfolgt dann eine Messung der Veränderung der Durchbiegung der Membran aufgrund der wirkenden Zellkraft. Aus der Höhenänderung der Membrandurchbiegung wird dann die Spannung in der Zellschicht berechnet. Hierbei vermindert sich die Durchbiegung der Membran aufgrund der Zellkraft, wenn die Zellen kontrahieren, d.h. die Füllstandshöhe der Flüssigkeitssäule bzw. des Kulturmediums wird geringfügig angehoben. Bei der Relaxation der Zellen (und/oder der extrazellulären Matrix) liegt der umgekehrte Fall vor. Diese Veränderung der Membrandurchbiegung wird dann erfindungsgemäß quantitativ und bevorzugt kontinuierlich mit Hilfe eines Sensors erfaßt.

Eine Variante der vorliegenden Erfindung umfaßt ein Verfahren zur Messung von Kräften von belebtem Material, wobei die durch das belebte Material auf die Membran übertragenen Kräfte als Veränderung der Füllstandshöhe der über der Membran befindlichen Flüssigkeitssäule gemessen werden. Hierbei wird die Durchbiegung der Membran als Regelgröße genutzt. Die durch die Kräfte des belebten Materials bewirkte Veränderung (Anhebung oder Absenkung der Füllstandshöhe) der über der Membran befindlichen Flüssigkeitssäule wird durch Zugabe oder Abführen einer entsprechenden Menge an Flüssigkeit derart kompensiert, daß die Durchbiegung der Membran den gleichen Wert beibehält, den sie vor der Übertragung der Kräfte des belebten Materials auf die Membran hatte. D.h. daß keine Veränderung der Durchbiegung der Membran meßbar ist bzw. die Membrandurchbiegung konstant ist oder gehalten wird.
Durch die genaue Bestimmung der zu- oder abgeführten Flüssigkeitsmenge kann die auf die Membran übertragene Kraft des belebten Materials bestimmt werden. Die entsprechenden Formeln und Umrechnungsfaktoren hierzu sind dem Fachmann bekannt und werden nicht weiter aufgeführt. Diese Vorgehensweise ist sowohl für die Bestimmung der Kräfte von belebtem Material geeignet, die auf einer Kontraktion des belebten Materials als auch auf einer Relaxation des belebten Materials beruhen.
Erfindungsgemäß umfaßt ist dabei ebenfalls eine geeignete Vorrichtung zur Messung der Veränderung der Füllstandhöhe der über der Membran befindlichen Flüssigkeitsmenge. Gegebenenfalls enthält diese Vorrichtung gegenüber der zuvor bereits beschriebenen Vorrichtung geeignete Komponenten zur automatisierten Entnahme oder Zuführung von Flüssigkeitsmengen, die ggf. mit dem Sensor kommunizieren, so daß eine Regelung gewährleistet ist, um die eingangs eingestellt Druchbiegung der Membran beizubehalten.

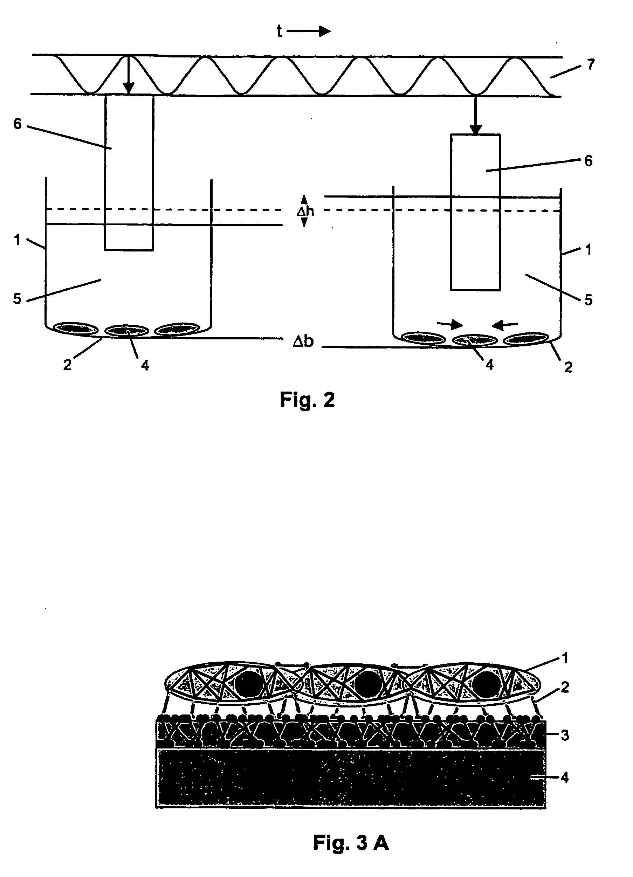

In einer besonderen Ausführungsvariante des erfindungsgemäßen Verfahrens wird das belebte Material externen mechanischen, elektrischen und/oder magnetischen Kräften ausgesetzt und so eine Zellkraftantwort stimuliert. Bevorzugt zeichnet sich das Verfahren dadurch aus, daß das belebte Material externen hydrostatischen Druckveränderungen ausgesetzt wird, indem ein Stempel zyklisch und mit veränderbarer Amplitude und Frequenz in die Flüssigkeitssäule über der Membran eingetaucht wird. Eine schematische Darstellung der Vorgehensweise ist in Fig. 2 gezeigt. Tieferes Eintauchen des Stempels in die Lösung über der Membran bewirkt einen höheren hydrostatischen Druck, flacheres Eintauchen bewirkt einen geringeren Druck. Die Druckveränderungen können aber auch durch das Anlegen eines Unterdrucks an die Membran der erfindungsgemäßen Vorrichtung erzeugt werden. Alternativ kann anstelle eines mechanischen Stempels ein magnetisches oder elektrisches Feld auf das belebte Material gerichtet werden. Das Anlegen der externen Kräfte kann sowohl während der Zellkultivierung, beispielsweise in einem Kulturschrank, als auch nach der Kultivierung im Meßgerät selbst erfolgen. Innerhalb der erfindungsgemäßen Vorrichtung ist die Halterung mit der Membran und dem belebten Material von dem Sensorbestandteil der erfindungsgemäßen Vorrichtung trennbar, so daß die Kultivierung und ggf. das Zelltraining im Kulturschank erfolgen und anschließend die so vorbereitete Halterung in die Vorrichtung eingesetzt wird, um die Messung mittels des zuvor genannten Sensors durchzuführen.

Durch die auftretenden sehr kleinen Dehnungen und Stauchungen wird das belebte Material mechanisch belastet und reagiert gegenüber einem unbelasteten Zustand mit einem veränderten Zell- und Matrixwachstum und/oder einer veränderten Genexpression. Das belebte Material erfährt durch das erfindungsgemäße Verfahren sozusagen einen "Trainingseffekt". Wesentlich für die vorliegende Erfindung ist, daß die Auswirkungen dieses "Trainings" direkt quantitativ durch die Messung der Zellkräfte bestimmt werden können. D. h., die Zellen werden zunächst trainiert und direkt im Anschluß daran, d.h. in ein und demselben Versuchsansatz, werden die Zellkräfte der trainierten Zellen gemessen. Dies stellt einen entscheidenden Vorteil gegenüber den bisher bekannten Verfahren zum Zelltraining dar. Bislang konnten nämlich mit den bekannten Zelldehnungsanordnungen lediglich Effekte, wie z. B. die Genexpression, der Calciumionenfluß über den Zellmembranen oder die Proteinsekretion bestimmt werden (Tschumperlin, D.J. et al., Deformationinduced injury of alveolar epithel cells, effect of frequency, duration and amplitude, Am. J. Respir. Crit Care Med, August 01, 2000, 162 (2): 357-362 und Hipper, A. et al., Cyclic mechanical strain decreases in the DNA synthesis of vascular smooth muscle cells, Pflugers Arch., May 2000, 44 (1): 19-27), nicht aber die Folgen der zellulären Kraftentwicklung, wie es durch die vorliegende Erfindung nun möglich ist.

In einer weiteren Variante zeichnet sich das erfindungsgemäße Verfahren dadurch aus, daß das belebte Material einem externen Zusatz chemischer und/oder biochemischer und/oder biologischer Verbindungen, bevorzugt in Form einer wässrigen Lösung, ausgesetzt wird. Hierbei wird der hydrostatische Druck über der Membran konstant gehalten, während der Zugabe von wässrigen Lösungen enthaltend chemische und/oder biochemische und/oder biologische Verbindungen gleichzeitig eine entsprechende Menge an bereits vorhandener wässriger Lösung über der Membran abgezogen wird.

Die in der wässrigen Lösung als "chemische Reize" bevorzugt enthaltenen chemischen und/oder biochemischen Verbindungen sind beispielsweise Thrombin, Trypsin, EDTA, Collagenase und/oder Kombinationen davon. Ferner sind als chemische Stimulanzien auch pharmakologisch wirksame Verbindungen, wie z. B. Inositol-Triphosphat IP3, Nocadazol/Taxol, Cytocalasin D, Cacium/Calmodulin, Fibronectin/Cycloheximid zu nennen. Außerdem können die Zellen durch Stickstoffmonoxid (NO) oder einen veränderten Sauerstoffpartialdruck stimuliert werden. Unter einer biologische Verbindung ist z. B. genetisches Material zu verstehen, durch das Veränderungen der Genexpression erzielt werden können. Ferner ist auch eine Stimulierung der Zellschichten, insbesondere der Proteinanteile (z. B. der interzellulären und/oder der Zellmatrix-Adhäsionsproteine und/oder der Zytoskelettproteine) durch Wechselwirkungen mit spezifischen Antikörpern denkbar. Dadurch kann u. a. der Beitrag einzelner Proteine an der zellulären Kraftentwicklung und/oder - weiterleitung untersucht werden. Ebenso können die auf der Membran wachsenden Zellschichten genetisch manipuliert sein und/oder werden. Durch gezielte Veränderungen des Kulturmediums kann dann z. B. die Genexpression stimuliert werden, so daß der Einfluß einzelner Genaktivitäten auf die Zellkräfte gezielt untersucht werden kann. Dies ist insbesondere bei der Entwicklung von Pharmaka von großem Interesse. Die zuvor gemachten Aufzählungen an Stimulanzien dienen lediglich zur Erläuterung der vorliegenden Erfindung, wirken sich jedoch nicht limitierend darauf aus.

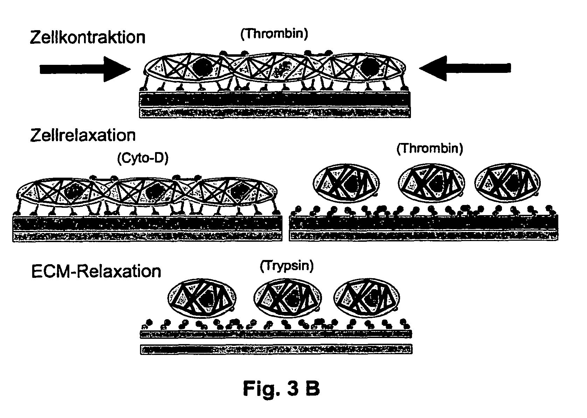

Der Effekt externer chemischer Reize auf die intrinsischen lateralen Kräfte des belebten Materials ist am Beispiel von Thrombin, EDTA und Trypsin in Fig. 3 schematisch dargestellt. Thrombin bewirkt dabei eine durch die Pfeile angedeutete Kontraktion des belebten Materials, Cytochalasin-D und EDTA bewirken eine Relaxation der Zellspannung aufgrund der Zerstörung des Cytoskeletts der Zellen bzw. aufgrund der Ablösung der Zellen von der extrazellulären Matrix (EZM). Trypsin schließlich bewirkt eine Relaxation der extrazellulären Matrix durch eine teilweise Degradation der extrazellulären Matrix und Ablösung von der Membran.

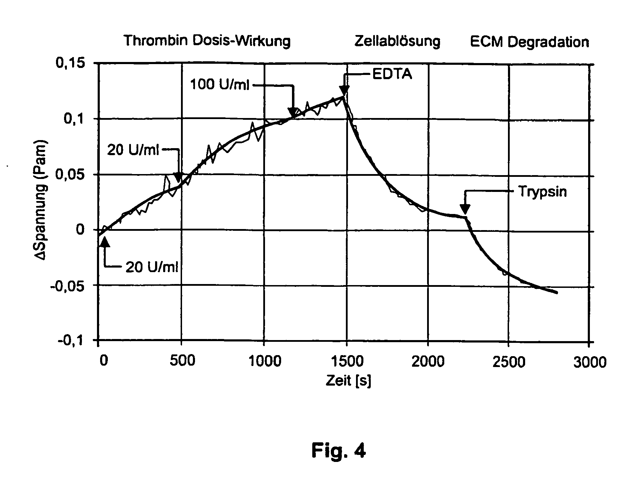

Fig. 4 zeigt eine Veränderung der Zellschichtspannung in Abhängigkeit von der Zeit unter Einwirkung von Thrombin, EDTA und Trypsin. Mit steigender Thrombinkonzentration ist hier ein Anstieg der Kontraktion des belebten Materials zu beobachten, wie der exponentielle Anstieg der Zellspannung zeigt. Werden mit EDTA die Verbindungen zwischen den Zellen und der extrazellulären Matrix gelöst, kann keine Kraftweiterleitung an die Membran erfolgen und die gemessenen intrinsischen Kräfte nehmen ab. Die verbleibende Restkraft, die von der extrazellulären Matrix ausgeht, nimmt ebenfalls ab, sobald diese Schicht durch die Zugabe von Trypsin weiter abgebaut wird.

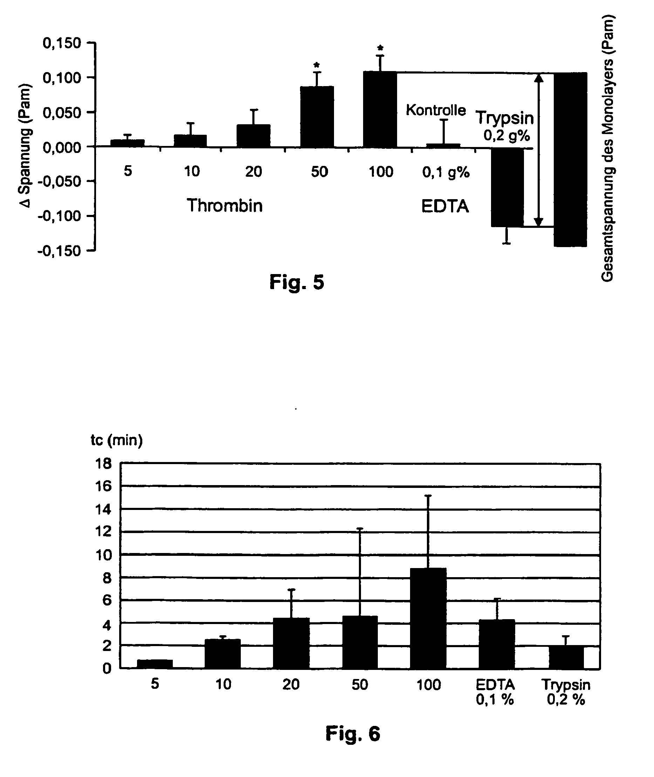

Dies zeigt deutlich, daß mit dem erfindungsgemäßen Verfahren nicht nur die absoluten oder nach erfolgter Stimulation erreichten Veränderungen der intrinsischen Kräfte gemessen werden können, sondern auch Reizleitungs- und Verzögerungszeiten. Die Messung der lateralen intrinsischen Kräfte des belebten Materials, insbesondere nach erfolgter chemischer oder mechanischer Reizung, ist aus physiologischen Gründen ein zeitabhängiger Prozeß, dessen Verlauf erfindungsgemäß kontinuierlich meßbar ist. Aus den erfindungsgemäßen Ergebnissen können Rückschlüsse auf die Zellkraft selbst, die Geschwindigkeit der Krafterzeugung oder auf die Kraftrelaxation nach Wegnahme des externen Reizes gezogen werden. Dies ist in Fig. 5 und Fig. 6 noch einmal dargestellt. Durch Thrombin nimmt die auf die Membran übertragene Spannung deutlich zu. Durch die Zugabe von EDTA erfolgt eine Relaxation der Membran bedingt durch die Ablösung der Zellen von der extrazellulären Matrix. Die Zugabe von Trypsin bewirkt ein Nachlassen der Spannung der extrazellulären Matrix durch deren Degradation.

Somit eignet sich die vorliegende Erfindung zur Messung von lateralen Kräften sowohl der Zellen mitsamt ihrer extrazellulären Matrix (EZM), die ursprünglich durch die Zellen sekretiert wird. Ebenso ist durch die vorliegende Erfindung die Messung der lateralen Kräfte auf die Membran nach Ablösung der Zellen von der EZM möglich, so daß auf diese Weise die Kräfte der EZM alleine meßbar sind. Darüber hinaus kann auch die extrazelluläre Matrix, beispielsweise enzymatisch, abgebaut werden und die verbleibenden Spannungen der Membran alleine gemessen werden. Auf diese Weise ist die konkrete Bestimmung der Kräfte möglich, die zu Beginn der Messungen durch das Überschichten der Membran mit einer wässrigen Lösung entsteht noch bevor belebtes Material auf die Membran aufgebracht worden ist (Vorspannung der Membran). Diese Werte sind als Eichwerte der vorgespannten Membran vor dem Beginn der Messung der lateralen Kräfte des belebten Materials anzusehen. Somit ist die in der erfindungsgemäßen Vorrichtung enthaltene Membran mechanisch genau charakterisiert.

Ganz besonders vorteilhaft an der vorliegenden Erfindung ist, daß sie alle nachfolgenden Eigenschaften gleichzeitig auf sich vereint.
Die erfindungsgemäße Vorrichtung und das erfindungsgemäße Verfahren ermöglichen die Untersuchung von Zellen im Zellverband, wobei die Zellzahl direkt sichtbar und zählbar ist. Auch das Cytoskelett ist anfärbbar und kann so sichtbar gemacht werden. Die Geometrie und mögliche Artefakte der Zellbewachsung der Membran sind erkennbar. Die Zellen bilden während des Wachstums eine extrazelluläre Matrix aus, die nach Ablösung von den Zellen getrennt physikalisch, chemisch und/oder mechanisch untersucht werden kann. Die Dicke der Zellschicht (z-Richtung) ist sehr gering (wenige µm) und bei dem erfindungsgemäßen Verfahren sehr klein gegenüber der Länge (in x- und y-Richtung; ca. 10 mm). Daraus folgt, daß der zu messende Spannungszustand der Zellschicht annäherunsgweise als zweidimensional betrachtet werden kann. Die Spannungszustände in der Membran selbst können genau definiert werden (Eichung). Weiterhin vereinigt die vorliegende Erfindung die folgenden Vorteile auf sich, wie eine Auflösung der Kraftmessung im Bereich von Nano- bzw. Pico-Newton, zeitliche Auflösung im Bereich von Millisekunden, hohe Meßgenauigkeit mit einer Fehlergrenze kleiner als 10 %, Messung absoluter und (chemisch und/oder physikalisch) stimulierter Kräfte der belebten Materie in einem Ansatz, Untersuchung hoher Zelldichten mit einer Zellzahl im Bereich von größer 1000 Zellen, sowie niedriger und routinetauglicher Präparationsaufwand bei geringem Kostenaufwand.
Erfindungsgemäß zeichnet sich das vorliegende Verfahren ferner dadurch aus, daß der zeitliche Verlauf der die auf die Membran übertragenen Kräfte, bevorzugt kontinuierlich, gemessen werden kann. Dieser kann über einen Zeitraum von 1 Sekunden bis hin zu mehreren Stunden erfolgen und ist insbesondere von der Lebensdauer der Zellen abhängig.
Die Abtastrate (definiert als die Anzahl der Meßpunkte pro Zeit) kann bei etwa 100/Sekunde liegen und ist zu kleineren Abtastraten (Untergrenze) hin unbegrenzt. Eine sinnvolle Untergrenze für die Abtastrate ist dabei 1/Minute.
Hierbei ist anzumerken, daß auch über einen langen Meßzeitraum der osmotische Druck der Flüssigkeit über der Membran konstant gehalten wird, und zwar durch Zugabe einer entsprechenden Menge an Flüssigkeit, die beispielsweise durch Verdunstung verloren gegangen ist. In einer weiteren Variante der vorliegenden Erfindung werden die von dem belebten Material ausgehenden intrinsischen Kräfte und/oder Kraftveränderungen (Linienlast und/oder Linienlastveränderungen) in einem Bereich von 0 bis 5000 mPa · m, bevorzugt von 0 bis 500 mPa · m gemessen. Ferner erfolgt die Messung der Kräfte und/oder Kraftveränderungen durch eine berührungslose Abtastung der Membran. Beispiele für berührungslose und rückwirkungsfreie Messmethoden sind die Abtastung der Membranverformung durch Laserstahlen, Interferenztechnologie, Atomic Force Mikroskopie oder Rastertunnel-Mikroskopie. Diese Aufzählung dient der Erläuterung und stellt keine Limitierung der vorliegenden Erfindung dar.

Gegenstand der vorliegenden Erfindung ist ferner ein Verfahren zur Identifizierung von Verbindungen (sogenanntens "Screening-Verfahren"), welche eine meßbare Wirkung auf die intrinsischen Kräfte von belebtem Material ausüben, wobei die Verbindungen in einem erfindungsgemäßen Verfahren der zuvor beschriebenen Art dem belebten Material zugesetzt werden und anschließend das Ausmaß der Veränderung der intrinsischen Kräfte unmittelbar bestimmt werden kann. So kann direkt gemessen werden, ob und in welchem Ausmaß, z.B. in Abhängigkeit von der eingesetzten Konzentration, die eingesetzten Verbindungen einen Effekt auf die Zellkräfte ausüben.

Gegenstand der vorliegenden Erfindung ist ferner die Verwendung einer Vorrichtung zur Messung von lateralen intrinsischen Kräften in belebtem Material, insbesondere von Zellschichten und/oder Organen/Organteilen. Hierbei sind auch kultivierte umfaßt. D. h. Gegenstand der vorliegenden Erfindung ist die Verwendung einer Vorrichtung enthaltend eine Halterung, eine elastisch verformbare Membran und einen Sensor zur Messung der Änderung von auf die Membran wirkenden Kräften, umfassend eine an einer Halterung angeordnete Membran, die plan gespannt, beidseitig frei zugänglich und durch Anlegen einer physikalischen Kraft elastisch verformbar ist und mit dem belebten Material verbindbar ist, nachdem es auf diese Membran aufgebracht ist.

Unter den zuvor genannten Kräften ist im Sinne der Erfindung die Linienlast (gemessen in Pa · m) zu verstehen. Sie hat die Dimension einer Kraft pro Länge. Diese Einheit folgt aus der Tatsache, daß die Zellschicht dünn ist im Vergleich zu ihrer lateralen Ausdehnung und daher in guter Annäherung als unendlich dünne Schicht betrachtet werden kann.

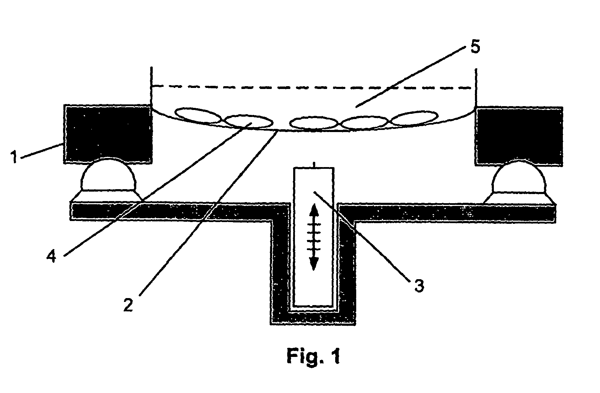

In einer besonderen Ausführungsvariante der vorliegenden Erfindung wird die elastisch verformbare Membran während eines Aufklebeprozesses derart über eine kreisförmige Halterung, z. B. die Unterseite eines Zylinders gezogen, daß die Membran völlig eben (plan) ist. Die Vorspannung wird so gewählt, daß die Membran eben ist, aber nicht plastisch verformt wird. Die Membran ist auch dann noch eben und nicht plastisch verformt, wenn zusätzliche Spannungen, z. B. durch auf die Membran aufgebrachtes Kulturmedium oder lebendes Material, auf die Membran einwirken. Eine schematische Darstellung der Vorrichtung ist in Fig. 1 skizziert.

Ferner umfaßt die erfindungsgemäß verwendete Vorrichtung eine Membran, die eine hydrophile Oberfläche und/oder eine zur Adhäsion und/oder Kultivierung von belebtem Material geeignete Oberfläche aufweist und/oder entsprechend behandelt und/oder mit einer haftvermittelnden Substanz beaufschlagt ist.
Da das belebte Material aufgrund seines Protein- und Kohlenhydratanteils hydrophil ist, sollte auch die in der erfindungsgemäßen Vorrichtung eingesetzte Membran hydrophil sein oder durch geeignete Maßnahmen entsprechend modifiziert bzw. aufbereitet werden. Dies kann z. B. durch eine Oberflächen-Plasmaätzung zur Erzeugung polarer Gruppen nach bekannten Methoden oder eine Beaufschlagung der Membranoberfläche mit haftvermittelnden (Kleber)-Substanzen, wie beispielsweise Gelatine, erreicht werden.

Darüber hinaus weist die erfindungsgemäß verwendete Membran eine Reihe weiterer Eigenschaften auf. Beispielsweise ist sie biokompatibel, nicht zytotoxisch, beständig gegen Stoffwechselprodukte und Umgebungsbedingungen des belebten Materials (biologisch inert), durchsichtig, hitze- und druckbeständig (autoklavierbar), reißfest, beständig gegenüber Säuren, Basen oder organischen Lösungsmitteln und weist eine niedrige Gasdurchlässigkeit auf. Die erfindungsgemäße Vorrichtung umfaßt dabei eine Membran, die enzymatisch abbaubares Material enthält. Dabei können z. B. Collagen, Elastin, Fibrinogen und/oder Kombinationen davon in und/oder auf die Membran ein- und/oder aufgebracht sein. Diese Aufzählung ist jedoch nicht limitierend für die vorliegende Erfindung.
Ferner umfaßt die erfindungsgemäße Vorrichtung eine Membran, die porenfrei ist und/oder eine Dicke im Bereich von 0,1 bis 10 µm, bevorzugt von 0,5 bis 5 µm und besonders bevorzugt von 1 µm aufweist, wobei das Verhältnis der Membrandicke zum Durchmesser oder Umfang oder der Kantenlänge der Membran (je nach dem, welche Form die Membran aufweist) einen Wert im Bereich von 6x10⁻⁶ bis 6x10⁻⁴, bevorzugt 3x10⁻⁵ bis 3x10⁻⁴ und besonders bevorzugt von 6x10⁻⁵ aufweist. Hierbei kann die Membran der erfindungsgemäßen Vorrichtung eine beliebige Form aufweisen, bevorzugt kreisförmig, halbkugelförmig, kugelförmig, rechteckig oder quadratisch. Außerdem ist die Membran mechanisch stabil und elastisch verformbar. Die erfindungsgemäße Vorrichtung zeichnet sich darüber hinaus dadurch aus, daß das Elastizitätsmodul der Membran bei 25°C einen Wert im Bereich von etwa 1000 bis 10000 MPa, bevorzugt von etwa 2500 bis 6500, besonders bevorzugt von etwa 3900 MPa aufweist.
In einer besonders bevorzugten Variante der vorliegenden Erfindung handelt es sich um eine Polyethylenfolie (PET). Denkbar ist ferner jegliches Biomaterial, insbesondere erprobte Biomaterialien für den Einsatz als Gefäßprothesen. Diese Ausgestaltungen sind jedoch nicht limitierend für die vorliegende Erfindung.

Die zuvor beschriebene erfindungsgemäß verwendete Vorrichtung zeichnet sich im weiteren dadurch aus, daß das auf die Membran aufgebrachte belebte Material ganze Zellen, eine oder mehrere Zellschicht(en) (Mono- oder Multilayer), sekretiertes Zellmaterial, bevorzugt extrazelluläre Matrix (EZM), Zellbestandteile und/oder Matrixbestandteile enthält, wobei das belebte Material genetisch verändert sein kann. In einer besonderen Ausführungsvariante der vorliegenden Erfindung handelt es sich bei dem belebten Material um Fibroblasten und/oder Muskelzellen, bevorzugt glatte Muskelzellen und/oder Endothelzellen, um nur eine nicht limitierende Auswahl zu nennen.

Ferner umfaßt die vorliegende Erfindung die Verwendung der beschriebenen Vorrichtung zur Identifizierung von chemischen und/oder biochemischen und/oder biologischen Verbindungen, beispielsweise von genetischem Material und/oder spezifischen Antikörpern, welche die lateralen intrinsischen Kräfte von belebtem Material, insbesondere Zellschichten und/oder Organen/Organteilen, beeinflussen.

Die Verwendung der erfindungsgemäß identifizierten Verbindungen ist insbesondere zur Herstellung von Mitteln zum Einsatz in Bereichen der Phamakologie bzw. Toxikologie und/ oder Transplantationsmedizin von großem Interesse.

Die vorliegende Erfindung wird durch die nachfolgenden Ausführungsbeispiele näher charakterisiert, die jedoch nicht limitierend sind:

### Herstellung der Meßvorrichtung und Aufbringung von belebtem Material:

Eine handelsübliche PET-Membran wird an einer offenen Seite eines Zylinders aufgeklebt, die andere Seite bleibt offen. Während des Aufklebeprozesses wird mit einem Metallring gleichmäßig an der Membran gezogen, so daß die Membran anschließend völlig eben gespannt ist, aber nicht plastisch verformt wird. Auf die Membran (mit einem Durchmesser von 16 mm) werden dann 400 µl Zellkulturmedium (DPBS) aufgebracht, entsprechend einer Füllstandshöhe von etwa 2 mm Flüssigkeitssäule. Die Membran ist aufgrund des Kulturmediums elastisch vorgeformt, d. h. durchgebogen und zur Aufbringung von belebtem Material vorbereitet.
Anschließend werden 1 ml einer Suspension von Fibroblasten (oder glatten Muskelzellen) mit einer Zellzahl von 5x10⁴/ml (oder 7x10⁴/ml) auf die vorgespannte Membran gegeben und die gesamte Vorrichtung zur Kultivierung der Zellen für 4 Tage bei 37 °C im Brutschrank inkubiert. Nach der Kultivierung wird das Kulturmedium gegen 400 µl frisches Medium ausgetauscht. Die so vorbereitete Halterung mit Membran und darauf kultivierten Zellen wird in die Meßvorrichtung eingesetzt und die Messung mittels eines Laserstrahls als Sensor durchgeführt.

Durch die Vermehrung haften die Zellen an der Membran an und bilden einen konfluenten Monolayer (oder Multilayer). Die Zellen sekretieren dabei mit der Zeit eine extazelluläre Matrix (EZM).
Aufgrund des Wachstums der Zellen und der damit verbundenen Zugspannung in der Zellschicht sowie in der ECM und der Tatsache, daß Zellen und die ECM an der Membran haften, erfährt die Membran eine Spannungsänderung. Infolge dessen verringert sich die Durchbiegung der Membran und hebt den Füllstand des Medium geringfügig an. Diese Veränderung der Durchbiegung der Membran wird mit Hilfe eines Lasers bestimmt.

### Stimulierung von belebtem Material:

Zur Stimulierung der Zellschicht(en) wird Thrombin in Endkonzentrationen von 0-100 U/ml zum Medium zugegeben. Zunächst erfolgt die Zugabe von 20 U/ml Kulturmedium. Das Thrombin bindet an die Thrombin-Rezeptoren und induziert eine Zellkontraktion. Die Membran hebt sich geringfügig weiter an. Dabei kontrahieren die Zellen mit einer typischen Zeitkonstante. Die gemessene Veränderung der Membrandurchbiegung erreicht nach einer theoretisch unendlichen Meßzeit einen typischen Sättigungswert. Successive werden weitere Thrombinmengen dem Zellkulturmedium über der Membran zugegeben und zwar in aufsteigender Reihenfolge, d. h. zuerst 50 U/ml und dann 100 U/ml. Entsprechend werden die Veränderungen der Membrandurchbiegung gemessen.
Es wird den Zellen jeweils erlaubt, die Kontraktion soweit auszubilden, daß im einfachsten Fall über das "Fitten einer Exponentialfunktion" das Erreichen des Endwertes nicht abgewartet werden muß, sondern das endgültige Maß der erreichbaren Kontraktion approximiert werden kann. Das verringert die Meßzeit. Aus der gefitteten Exponentialfunktion kann sowohl die charakteristische Einstellzeit der Trombin-vermittelten Kontraktionswirkung errechnet werden als auch die bei dieser Kontraktion erreichbare maximale Kontraktion.

### Sukkzessive Ablösung des belebten Materials und der EZM von der Membran (Eichung):

Durch die Zugabe von EDTA (0,1 Gew.-%) über eine Einwirkzeit von 2 min werden die Zellen von der EZM abgelöst. Die zugegebenen Flüssigkeitsmengen werden dabei an anderer Stelle der Meßkammer gleichzeitig mit gleichem Volumen wieder entnommen. Die Zellen lösen sich mit einer typischen Zeitkonstante ab. Als Folge davon verändert sich die Spannung in der Membran, da die durch die Zellkontraktion auf die Membran wirkenden Kräfte entfallen, so daß die Membran sich wieder weiter durchbiegt. Auch hier wird eine Exponentialfunktion approximiert und es können Zeitkonstante und der Endwert der Spannungserholung approximativ berechnet werden.

Die auf der Membran haftende EZM hält noch einen Teil der während des Zellwachstums entstandenen Spannung aufrecht. Durch die Zugabe von Trypsin (0,2 Gew.-%) über eine Einwirkzeit von 15 min wird die EZM degradiert. Auch wird wird gleichzeitig die gleiche Flüssigkeitsmenge wieder entnommen. Dabei werden die von Trypsin degradierbaren Proteine der EZM zerstört. Die Membran sinkt weiter durch. Die Veränderung der Membrandurchbiegung wird gemessen.

Der noch auf der Membran verbleibende Rest der EZM, der durch Trypsin nicht degradiert wurde, wird im folgenden durch Collagenase abgebaut. Dem können sich weitere Degradationsprozesse anschließen, so daß keine Matrix mehr auf der Membran verbleibt. Die Membran befindet sich nach dieser(n) Degradation(en) in etwa dem gleichen Spannungszustand, den sie hatte, bevor die Zellen auf der Membran konfluent angewachsen waren und die Matrix ausgebildet worden war.

Zur Ermittlung der Vorspannung der Membran (Eichung) wird nun durch Zugabe von DPBS der hydrostatische Druck über der Membran erhöht. Die zugegebene Menge an DPBS beträgt etwa 8 µl, wodurch sich die Membran in der Mitte um etwa 40µm weiter durchbiegt. Aus diesem sogenannten "Eich-Drucksprung" und der gemessenen Durchbiegungsänderung wird die Vorspannung der Membran ermittelt Da bei jedem vorangegangenen Schritt ab dem Zeitpunkt des Einbringes der Zellen die jeweilige Durchbiegung der Membran gemessen wurde, kann nun direkt auf die von den Zellen erzeugte Spannung, auf die durch Thrombin erzeugte Spannungsänderungen der Zellen und deren Zeitkonstante, auf die durch Trypsin erzeugte Spannungsänderung der EZM (EZM Degradation) und deren Zeitkonstanten etc. zurückgerechnet werden.

Der jeweilige Durchbiegungszustand der Membran wird mit einem Laserstrahl, der von unten, d.h. außerhalb der Vorrichtung, flach auf die Membranmitte gerichtet ist und dort reflektiert wird, gemessen. Der reflektierte Strahl wird von einer Vierquadrantendiode erfaßt. Die Verschiebung der Intensitätsverteilung zwischen den Quadranten führt zu einer exakten Berechenbarkeit der Durchbiegung der Membran. Die während den Messungen, die z. T. einige Stunden dauern können, auftretenden Verdunstungen geringer Mengen an wässriger Flüssigkeit über der Membran, die sich als Änderung der Durchbiegung der Membran bemerkbar machen würden, wird durch externe Zugabe von Wasser zum Medium kompensiert. Dabei bleibt der osmotische Druck der Meßlösung während der Messung konstant.

### Legende zu den Figuren:

- Fig.: 1: Schematische Darstellung einer Vorrichtung zur Messung von Kräften von belebtem Material enthaltend eine Halterung (1), eine Membran (2) mit darauf aufgebrachtem belebtem Material (4) und einen Sensor (3). Die gestrichelte Linie stellt die Füllstandshöhe der Flüssigkeitssäule (5) über der Membran dar.
- Fig. 2:: Schematische Darstellung zur Vorgehensweise des "Trainings" von belebtem Material durch externe physikalische Reize. Durch das Eintauchen eines Stempels (6) in die Flüssigkeitssäule (5) über der Membran (2), die an einer Halterung (1) angebracht ist und auf der belebtes Material (4) aufgebracht ist, wird der Druck in der Vorrichtung erhöht. Das Senken (Heben) des Stempels um Δh führt zur Änderung der Durchbiegung der Membran um Δb, die ihrerseits zu einer Änderung der Zug- bzw. Druckspannung in dem belebten Material führt. Das Heben und Senken des Stempels kann während der Kultivierung des belebten Materials auf der Membran erfolgen und ist als eine Funktion der Zeit (t) beispielhaft in Form einer sinusförmigen Kurve (7) dargestellt.
- Fig. 3: A): Schematische Darstellung des belebten Material enthaltend Zellen (1), Adhäsionsmoleküle (2) und extrazelluläre Matrix (3) auf einer Membran (4).
B) Schematische Darstellung der Veränderungen des belebten Materials durch Zugabe externer chemischer Stimulanzien.
- Fig. 4:: Darstellung einer Dosis-Wirkungskurve von Thrombin, EDTA und Trypsin als Funktion der gemessenen intrinsischen Kräfte von belebtem Material (MPa) im zeitlichen Verlauf (s).
- Fig. 5:: Balkendiagramm als Darstellung der Veränderung der intrinsischen Kräfte (MPa) von belebtem Material in Anwesenheit von entsprechenden Mengen (U/ml) an Thrombin, EDTA und Trypsin.
- Fig. 6:: Balkendiagramm als Darstellung der Relaxationszeiten (min) von belebtem Material in Abhängigkeit von der Zugabe entsprechender Mengen (U/ml) an Thrombin, EDTA bzw. Trypsin.
- Fig. 7:: Darstellung der Eigenperistaltik (Eigenkontraktion und -relaxation) von Rinderaorten-Endothelzellen (RAEZ) als Funktion der gemessenen intrinsischen Kräfte von belebtem Material (MPa) im zeitlichen Verlauf (s), mit einer Periodendauer von 55s.

## Patentansprüche

1. Verfahren zur Messung von Kräften von belebtem Material, **dadurch gekennzeichnet, daß** lateral wirkende intrinsische Kräfte des belebten Materials, ggf. vor, während und/oder nach erfolgter Stimulation des Materials durch externe Reize, direkt auf eine elastisch verformbare Membran übertragen werden und die hieraus resultierende Änderung der Durchbiegung der Membran quantitativ erfaßt wird.

2. Verfahren gemäß Anspruch 1, **dadurch gekennzeichnet, daß**
a) eine elastisch verformbare Membran an einer Halterung plan gespannt wird, so daß sie von beiden Seiten frei zugänglich ist,
b) die Membran durch das Anlegen einer physikalischen Kraft elastisch verformt wird,
c) auf die elastisch verformte Membran belebtes Material aufgebracht und durch Kultivierung des belebten Materials und/oder eine Haftvermittelung in und/oder auf der Membran, eine Verbindung zwischen dem belebten Material und der Membran ausgebildet wird,
d) ggf. das belebte Material während und/oder nach der Ausbildung der Verbindung mit der Membran konstanten und/oder pulsierend und/oder schwingend angelegten externen Kräften ausgesetzt wird,
e) die von dem belebten Material ausgehenden lateralen intrinsichen Kräfte auf die Membran übertragen werden,
f) ggf. zusätzliche externe Reize auf das belebte Material ausgeübt werden und
g) die auf die Membran übertragenen Kräfte und/oder Kraftänderungen des belebten Materials, die damit verbundenen Zeitkonstanten und/oder Relaxationszeiten kontinuierlich als Veränderungen der Durchbiegung der Membran mit Hilfe eines Sensors quantitativ bestimmt werden.

3. Verfahren gemäß einem der Ansprüche 1 oder 2, **dadurch gekennzeichnet, daß** das auf die Membran aufgebrachte belebte Material ganze Zellen, eine oder mehrere Zellschicht(en), sekretiertes Zellmaterial, bevorzugt extrazelluläre Matrix, Zellbestandteile und/oder Matrixbestandteile enthält.

4. Verfahren gemäß einem der Ansprüche 1 bis 3, **dadurch gekennzeichnet, daß** die ganzen Zellen Fibroblasten und/oder Muskelzellen und/oder Endothelzellen sind.

5. Verfahren gemäß einem der Ansprüche 1 bis 4, **dadurch gekennzeichnet, daß** genetisch verändertes belebtes Material eingesetzt wird.

6. Verfahren gemäß einem der Ansprüche 1 bis 5, **dadurch gekennzeichnet, daß** Antikörper mit einer spezifischen Bindung an interzelluläre und/oder Zellmatrix-Adhäsionsproteine und/oder Zytoskelettproteine eingesetzt werden.

7. Verfahren gemäß einem der Ansprüche 1 bis 6, **dadurch gekennzeichnet, daß** die Membran während und/oder nachdem sie plan gespannt wird/ist mit der Halterung verklebt wird.

8. Verfahren gemäß einem der Ansprüche 1 bis 7, **dadurch gekennzeichnet, daß** die Membran durch die Überschichtung mit einer Flüssigkeitssäule elastisch verformt wird.

9. Verfahren gemäß einem der Ansprüche 1 bis 8, **dadurch gekennzeichnet, daß** an die Membran ein hydrostatischer Druck entsprechend einer Flüssigkeitssäule mit einer Füllstandshöhe im Bereich von etwa 0,1 bis 50 mm, bevorzugt von 0,5 bis 10 mm und besonders bevorzugt von 2 mm, bei einem Elastizitätsmodul von etwa 3900 MPa, angelegt wird.

10. Verfahren gemäß einem der Ansprüche 1 bis 9, **dadurch gekennzeichnet, daß** die Flüssigkeitssäule ein zur Kultivierung des belebten Materials geeignetes Medium ist.

11. Verfahren gemäß einem der Ansprüche 1 bis 10, **dadurch gekennzeichnet, daß** das belebte Material externen mechanischen, elektrischen und/oder magnetischen Kräften ausgesetzt wird.

12. Verfahren gemäß einem der Ansprüche 1 bis 11, **dadurch gekennzeichnet, daß** das belebte Material externen hydrostatischen Druckveränderungen ausgesetzt wird, indem ein Stempel zyklisch und mit veränderbarer Amplitude und Frequenz in die Flüssigkeitssäule über der Membran eingetaucht wird und/oder ein Unterdruck an die Membran angelegt wird.

13. Verfahren gemäß einem der Ansprüche 1 bis 12, **dadurch gekennzeichnet, daß** das belebte Material einem externen Zusatz chemischer und/oder biochemischer und/oder biologischer Verbindungen, bevorzugt in Form einer wässrigen Lösung, ausgesetzt wird.

14. Verfahren gemäß einem der Ansprüche 1 bis 13, **dadurch gekennzeichnet, daß** durch den Zusatz von wässrigen Lösungen enthaltend chemische und/oder biochemische und/oder biologische Verbindungen, der hydrostatische Druck über der Membran konstant gehalten wird, indem gleichzeitig eine entsprechende Menge an vorhandener wässriger Lösung über der Membran abgezogen wird.

15. Verfahren gemäß einem der Ansprüche 1 bis 14, **dadurch gekennzeichnet, daß** als chemische und/oder biochemische Verbindungen Thrombin, Trypsin, EDTA, Collagenase und/oder Kombinationen davon zugesetzt werden und/oder als biologische Verbindungen genetisches Material und/oder spezifische Antikörper zugesetzt werden.

16. Verfahren gemäß einem der Ansprüche 1 bis 15, **dadurch gekennzeichnet, daß** der zeitliche Verlauf der auf die Membran übertragenen Kräfte, bevorzugt kontinuierlich, über einen Zeitraum von 1 Sekunde bis mehrere Stunden gemessen werden.

17. Verfahren gemäß einem der Ansprüche 1 bis 16, **dadurch gekennzeichnet, daß** von dem belebten Material ausgehende intrinsische Kräfte und/oder Kraftveränderungen in einem Bereich von 0 bis 5000 mPa·m, bevorzugt von 0 bis 500 mPa·m gemessen werden.

18. Verfahren gemäß einem der Ansprüche 1 bis 17, **dadurch gekennzeichnet, daß** die Messung der Kräfte und/oder Kraftveränderungen durch eine berührungslose Abtastung der Membran erfolgt.

19. Verwendung einer Vorrichtung enthaltend eine Halterung, eine elastisch verformbare Membran und einen Sensor zur Messung der Änderung von auf die Membran wirkenden Kräften, umfassend eine an einer Halterung angeordnete Membran, die plan gespannt, beidseitig frei zugänglich und durch Anlegen einer physikalischen Kraft elastisch verformbar ist und mit dem belebten Material verbindbar ist, nachdem es auf diese Membran aufgebracht ist, zur Messung von lateralen intrinsischen Kräften von belebtem Material.

20. Verwendung gemäß Anspruch 19, **dadurch gekennzeichnet, daß** die Membran eine hydrophile Oberfläche und/oder eine zur Adhäsion und/oder Kultivierung von *belebtem Material geeignete Oberfläche aufweist und/oder entsprechend behandelt und/oder mit einer haftvermittelnden Substanz beaufschlagt ist.

21. Verwendung gemäß einem der Ansprüche 19 oder 20, **dadurch gekennzeichnet, daß** die Membran eine beliebige Form, bevorzugt kreisförmig, halbkugelförmig, kugelförmig, rechteckig und/oder quadratisch aufweist.

22. Verwendung gemäß einem der Ansprüche 19 bis 21, **dadurch gekennzeichnet, daß** die Membran porenfrei und/oder reißfest und/oder biologisch inert ist.

23. Verwendung gemäß einem der Ansprüche 19 bis 22, **dadurch gekennzeichnet, daß** die Membran eine Dicke im Bereich von 0,1 bis 10 µm, bevorzugt 0,5 bis 5 µm, besonders bevorzugt 1 µm aufweist, wobei das Verhältnis der Membrandicke zum Durchmesser oder Umfang oder der Kantenlänge der Membran einen Wert im Bereich von 6x10⁻⁶ bis 6x10⁻⁴, bevorzugt 3x10⁻⁵ bis 3x10⁻⁴ und besonders bevorzugt von 6x10⁻⁵ aufweist.

24. Verwendung gemäß einem der Ansprüche 19 bis 23, **dadurch gekennzeichnet, daß** das Elastizitätsmodul der Membran bei 25 °C einen Wert im Bereich von etwa 1000 bis 10000 MPa, bevorzugt von etwa 2500 bis 6500 MPa und besonders bevorzugt von etwa 3900 MPa aufweist.

25. Verwendung gemäß einem der Ansprüche 19 bis 24, **dadurch gekennzeichnet, daß** die Membran enzymatisch abbaubares Material enthält, welches in und/oder auf die Membran ein- und/oder aufgebracht ist.

26. Verwendung gemäß einem der Ansprüche 19 bis 25, **dadurch gekennzeichnet, daß** in und/oder auf die Membran Collagen, Elastin und/oder Fibrinogen und/oder Kombinationen davon ein- und/oder aufgebracht sein.

27. Verwendung gemäß einem der Ansprüche 19 bis 26 zur Identifizierung von chemischen und/oder biochemischen und/oder biologischen Verbindungen, welche die lateralen intrinsischen Kräfte von belebten Material beeinflussen.

## Claims

1. Method for measuring forces of living material, **characterized in that** laterally acting intrinsic forces of the living material are transmitted directly, where appropriate before, during and/or after stimulation of the material with external stimuli, to an elastically deformable membrane and the resulting change in the deflection of the membrane is registered quantitatively.

2. Method according to Claim 1, **characterized in that**
a) an elastically deformable membrane is stretched in a planar manner on a mounting such that it is freely accessible from both sides,
b) the membrane is elastically deformed by applying a physical force,
c) living material is applied to the elastically deformed membrane and a bond is formed between the living material and the membrane by means of culturing the living material and/or an adhesion mediation in and/or on the membrane,
d) where appropriate, the living material is subjected, during and/or after the formation of the bond with the membrane, to external forces which are applied constantly and/or in a pulsating manner and/or in an oscillating manner,
e) the lateral intrinsic forces which emanate from the living material are transmitted to the membrane,
f) where appropriate, additional external stimuli are exerted on the living material, and
g) the forces and/or force changes of the living material which have been transmitted to the membrane, and the time constants and/or relaxation times which are associated therewith, are quantitatively determined continuously, as changes in the deflection of the membrane, using a sensor.

3. Method according to either Claim 1 or 2, **characterized in that** the living material which is applied to the membrane contains whole cells, one or more cell layer(s), secreted cell material, preferably extracellular matrix, cell constituents and/or matrix constituents.

4. Method according to one of Claims 1 to 3, **characterized in that** the whole cells are fibroblasts and/or muscle cells and/or endothelial cells.

5. Method according to one of Claims 1 to 4, **characterized in that** genetically altered living material is employed.

6. Method according to one of Claims 1 to 5, **characterized in that** antibodies which bind specifically to intercellular and/or cell matrix adhesion proteins and/or cytoskeletal proteins are employed.

7. Method according to one of Claims 1 to 6, **characterized in that** the membrane is adhered to the mounting while it is being and/or after it has been stretched in a planar manner.

8. Method according to one of Claims 1 to 7, **characterized in that** the membrane is elastically deformed by being overlaid with a liquid column.

9. Method according to one of Claims 1 to 8, **characterized in that** a hydrostatic pressure, corresponding to a liquid column having a fill height in the range from about 0.1 to 50 mm, preferably of from 0.5 to 10 mm, and particularly preferably of 2 mm, is applied to the membrane, at a modulus of elasticity of about 3900 MPa.

10. Method according to one of Claims 1 to 9, **characterized in that** the liquid column is a medium which is suitable for culturing the living material.

11. Method according to one of Claims 1 to 10, **characterized in that** the living material is subjected to external mechanical, electrical and/or magnetic forces.

12. Method according to one of Claims 1 to 11, **characterized in that** the living material is subjected to external hydrostatic pressure changes by a plunger being immersed, cyclically and with changeable amplitude and frequency, into the liquid column over the membrane, and/or a negative pressure being applied to the membrane.

13. Method according to one of Claims 1 to 12, **characterized in that** the living material is subjected to an external addition of chemical and/or biochemical and/or biological compounds, preferably in the form of an aqueous solution.

14. Method according to one of Claims 1 to 13, **characterized in that**, as a result of adding aqueous solutions containing chemical and/or biochemical and/or biological compounds, the hydrostatic pressure above the membrane is kept constant by simultaneously withdrawing a corresponding quantity of aqueous solution which is present above the membrane.

15. Method according to one of Claims 1 to 14, **characterized in that** thrombin, trypsin, EDTA or collagenase and/or combinations thereof are added as chemical and/or biochemical compounds, and/or genetic material and/or specific antibodies are added as biological compounds.

16. Method according to one of Claims 1 to 15, **characterized in that** the time course of the forces which are transmitted to the membrane is measured, preferably continuously, over a period of from 1 second to several hours.

17. Method according to one of Claims 1 to 16, **characterized in that** intrinsic forces and/or force changes emanating from the living material are measured in a range from 0 to 5000 mPa·m, preferably from 0 to 500 mPa·m.

18. Method according to one of Claims 1 to 17, **characterized in that** the forces and/or force changes are measured by sampling the membrane in a manner which does not involve any contact.

19. Use of a device containing a mounting, an elastically deformable membrane and a sensor for measuring the change of forces acting on the membrane, comprising a membrane which is arranged on a mounting, which is stretched in a planar manner, which is freely accessible from both sides and which can be deformed elastically by applying a physical force, and which can be bonded to the living material after the latter has been applied to this membrane, for measuring lateral intrinsic forces of living material.

20. Use according to Claim 19, **characterized in that** the membrane exhibits a hydrophilic surface and/or a surface which is suitable for adhering and/or culturing living material, and/or is correspondingly treated and/or supplied with an adhesion-mediating substance.

21. Use according to either Claim 19 or 20, **characterized in that** the membrane has any arbitrary shape, being preferably circular, hemispherical, spherical, rectangular and/or square.

22. Use according to one of Claims 19 to 21, **characterized in that** the membrane is pore-free and/or tear-resistant and/or biologically inert.

23. Use according to one of Claims 19 to 22, **characterized in that** the thickness of the membrane is in the range from 0.1 to 10 µm, preferably from 0.5 to 5 µm, particularly preferably 1 µm, with the ratio of the thickness of the membrane to the diameter or circumference or edge length of the membrane having a value in the range from 6 × 10⁻⁶ to 6 × 10⁻⁴, preferably from 3 × 10⁻⁵ to 3 × 10⁻⁴, and particularly preferably 6 × 10⁻⁵.

24. Use according to one of Claims 19 to 23, **characterized in that** the value of the modulus of elasticity of the membrane at 25°C is in the range from about 1000 to 10 000 MPa, preferably from about 2500 to 6500 MPa, and particularly preferably about 3900 MPa.

25. Use according to one of Claims 19 to 24, **characterized in that** the membrane contains enzymically degradable material which is introduced and/or applied into and/or onto the membrane.

26. Use according to one of Claims 19 to 25, **characterized in that** collagen, elastin and/or fibrinogen and/or combinations thereof is/are introduced and/or applied into and/or onto the membrane.

27. Use according to one of Claims 19 to 26 for identifying chemical and/or biochemical and/or biological compounds which influence the lateral intrinsic forces of living material.

## Revendications

1. Procédé pour mesurer des forces produites par une matière vivante, **caractérisé en ce que** des forces intrinsèques issues de la matière vivante et s'exerçant latéralement, sont transmises, éventuellement avant, pendant et/ou après stimulation de la matière par le biais de stimuli externes, directement à une membrane élastiquement déformable et **en ce que** l'on enregistre quantitativement la variation résultante du fléchissement de la membrane.

2. Procédé selon la revendication 1, **caractérisé en ce que** :
a) on tend à plat une membrane élastiquement déformable sur un système de fixation, de sorte que celle-ci soit accessible librement des deux côtés,
b) on soumet la membrane à une déformation élastique en appliquant une force physique,
c) on applique une matière vivante sur la membrane déformée élastiquement et on laisse s'établir une connexion entre la matière vivante et la membrane en procédant à la culture de la matière vivante et/ou par adhérence de cette dernière dans et/ou sur la membrane,
d) on expose éventuellement la matière vivante pendant et/ou après que la connexion a été établie avec la membrane, à des forces externes que l'on applique de manière constante et/ou selon un mode d'impulsions et/ou d'oscillations,
e) les forces intrinsèques latérales provenant de la matière vivante sont transmises à la membrane,
f) on exerce, éventuellement, des stimuli externes supplémentaires sur la matière vivante, et
g) on détermine quantitativement et en continu les forces transmises à la membrane et/ou les variations de force de la matière vivante, les constantes de temps et/ou les temps de relaxation qui y sont associés, comme modifications du fléchissement de la membrane, à l'aide d'un capteur.

3. Procédé selon l'une quelconque des revendications 1 ou 2, **caractérisé en ce que** la matière vivante appliquée sur la membrane contient des cellules entières, une ou plusieurs couches de cellules, un matériau cellulaire secrété, de préférence, une matrice extracellulaire, des composants cellulaires et/ou des composants de matrice.

4. Procédé selon l'une quelconque des revendications 1 à 3, **caractérisé en ce que** les cellules entières sont des fibroblastes et/ou des cellules musculaires et/ou des cellules endothéliales.

5. Procédé selon l'une quelconque des revendications 1 à 4, **caractérisé en ce que** l'on utilise une matière vivante génétiquement modifiée.

6. Procédé selon l'une quelconque des revendications 1 à 5, **caractérisé en ce que** l'on utilise des anticorps présentant une liaison spécifique à des protéines d'adhérence intercellulaire et/ou de la matrice cellulaire et/ou à des protéines du cytosquelette.

7. Procédé selon l'une quelconque des revendications 1 à 6, **caractérisé en ce que** la membrane est collée au système de fixation pendant et/ou après qu'elle a été tendue à plat.

8. Procédé selon l'une quelconque des revendications 1 à 7, **caractérisé en ce que** la membrane subit une déformation élastique en la recouvrant d'une colonne de liquide.

9. Procédé selon l'une quelconque des revendications 1 à 8, **caractérisé en ce que** l'on applique sur la membrane une pression hydrostatique correspondant à une colonne de liquide ayant un niveau de liquide situé dans la plage d'environ 0,1 à 50 mm, de préférence, de 0,5 à 10 mm et, en particulier, de 2 mm, pour un module d'élasticité d'environ 3900 MPa.

10. Procédé selon l'une quelconque des revendications 1 à 9, **caractérisé en ce que** la colonne de liquide est un milieu convenant à la culture de la matière vivante.

11. Procédé selon l'une quelconque des revendications 1 à 10, **caractérisé en ce que** la matière vivante est exposée à des forces mécaniques, électriques et/ou magnétiques externes.

12. Procédé selon l'une quelconque des revendications 1 à 11, **caractérisé en ce que** la matière vivante est exposée à des variations de pression hydrostatique en immergeant un vérin plongeur de manière cyclique et selon une amplitude et une fréquence variables dans la colonne de liquide au-dessus de la membrane et/ou en appliquant une dépression sur la membrane.

13. Procédé selon l'une quelconque des revendications 1 à 12, **caractérisé en ce que** la matière vivante est exposée en ajoutant de manière externe des composés chimiques et/ou biochimiques et/ou biologiques, de préférence, sous la forme d'une solution aqueuse.

14. Procédé selon l'une quelconque des revendications 1 à 13, **caractérisé en ce que** l'addition de solutions aqueuses contenant des composés chimiques et/ou biochimiques et/ou biologiques, permet de maintenir la pression hydrostatique constante sur la membrane, tandis que l'on prélève simultanément une quantité correspondante de solution aqueuse présente sur la membrane.

15. Procédé selon l'une quelconque des revendications 1 à 14, **caractérisé en ce que** l'on ajoute, comme composés chimiques et/ou biochimiques, de la thrombine, de la trypsine, de l'EDTA, une collagénase et/ou leurs combinaisons et/ou, comme composés biologiques, un matériau génétique et/ou des anticorps spécifiques.

16. Procédé selon l'une quelconque des revendications 1 à 15, **caractérisé en ce que** l'évolution au cours du temps des forces transmises sur la membrane, sont mesurées, de préférence, en continu sur une période de temps s'étendant d'une seconde à plusieurs heures.

17. Procédé selon l'une quelconque des revendications 1 à 16, **caractérisé en ce que** des forces intrinsèques et/ou les variations de force provenant de la matière vivante, sont mesurées dans une plage de 0 à 5000 mPa·m, de préférence, de 0 à 500 mPa·m.

18. Procédé selon l'une quelconque des revendications 1 à 17, **caractérisé en ce que** la mesure des forces et/ou des variations de forces s'effectue par une exploration de la membrane sans contact avec celle-ci.

19. Utilisation d'un dispositif contenant un système de fixation, une membrane élastiquement déformable et un capteur destiné à mesurer la variation des forces s'exerçant sur la membrane, comprenant une membrane aménagée sur un système de fixation, laquelle est tendue à plat, accessible librement des deux côtés et élastiquement déformable par application d'une force physique, et qui peut se connecter à la matière vivante une fois celle-ci appliquée sur cette membrane, en vue de mesurer des forces intrinsèques latérales issues de la matière vivante.

20. Utilisation selon la revendication 19, **caractérisée en ce que** la membrane présente une surface hydrophile et/ou une surface convenant à l'adhérence et/ou à la culture de la matière vivante, et/ou est traitée de manière correspondante et/ou soumis à l'influence d'une substance adhésive.

21. Utilisation selon l'une quelconque des revendications 19 à 20, **caractérisée en ce que** la membrane présente une forme quelconque, de préférence, circulaire, hémisphérique, sphérique, rectangulaire et/ou carrée.

22. Utilisation selon l'une quelconque des revendications 19 à 21, **caractérisée en ce que** la membrane est exempte de pores et/ou résistante à la rupture et/ou inerte au plan biologique.

23. Utilisation selon l'une quelconque des revendications 19 à 22, **caractérisée en ce que** la membrane présente une épaisseur située dans la plage de 0,1 à 10 µm, de préférence, de 0,5 à 5 µm, mieux encore, de 1 µm, le rapport de l'épaisseur de la membrane au diamètre ou à la périphérie ou à la longueur d'arête de la membrane présentant une valeur située dans la plage de 6 x 10⁻⁶ à 6 x 10⁻⁴, de préférence, de 3 x 10⁻⁵ à 3 x 10⁻⁴ et, mieux encore, de 6 x 10⁻⁵.

24. Utilisation selon l'une quelconque des revendications 19 à 23, **caractérisée en ce que** le module d'élasticité présente, à 25 °C, une valeur située dans la plage d'environ 1000 à 10 000 MPa, de préférence, d'environ 2500 à 6500 MPa et, mieux encore, d'environ 3900 MPa.

25. Utilisation selon l'une quelconque des revendications 19 à 24, **caractérisée en ce que** la membrane contient un matériau dégradable par voie enzymatique, lequel est incorporé à la membrane et/ou appliqué sur celle-ci.

26. Utilisation selon l'une quelconque des revendications 19 à 25, **caractérisée en ce que** l'on incorpore à la membrane, et/ou **en ce que** l'on applique sur celle-ci, du collagène, de l'élastine et/ou du fibrinogène et/ou leurs combinaisons.

27. Utilisation selon l'une quelconque des revendications 19 à 26, pour identifier des composés chimiques et/ou biochimiques et/ou biologiques, qui influencent les forces intrinsèques latérales provenant d'une matière vivante.
